**Europäisches Patentamt**

**European Patent Office**

**Office européen des brevets**

(11) Publication number: **0 126 394**
**B1**

## EUROPEAN PATENT SPECIFICATION

(45) Date of publication of patent specification: **28.10.87**

(51) Int. Cl.⁴: **C 02 F 3/34**, C 12 N 9/02

(21) Application number: **84105336.6**

(22) Date of filing: **11.05.84**

(54) Enzymatic removal of aromatic hydroxy compounds and aromatic amines from waste waters.

(30) Priority: **13.05.83 US 494489**

(43) Date of publication of application:
**28.11.84 Bulletin 84/48**

(45) Publication of the grant of the patent:
**28.10.87 Bulletin 87/44**

(84) Designated Contracting States:
**AT BE CH DE FR GB IT LI LU NL SE**

(56) References cited:

CHEMICAL ABSTRACTS, vol. 95, no. 10, 7th
September 1981, page 296, no. 85697s,
Columbus, Ohio, USA; A.M. KLIBANOV et al.:
"Enzymic removal of toxic phenols and anilines
from waste waters"

CHEMICAL ABSTRACTS, vol. 96, no. 14, 5th
April 1982, page 346, no. 109578c, Columbus,
Ohio, USA; B.N. ALBERTI et al.: "Enzymic
removal of dissolved aromatics from industrial
aqueous effluents"

(73) Proprietor: **PHILLIPS PETROLEUM COMPANY**
**5th and Keeler**
**Bartlesville Oklahoma 74004 (US)**

(72) Inventor: **Hopkins, Thomas R.**
**3417 S.E. Willowood Dr.**
**Bartlesville Oklahoma 74006 (US)**

(74) Representative: **Dost, Wolfgang, Dr.rer.nat.,**
**Dipl.-Chem. et al**
**Patent- und Rechtsanwälte Bardehle-**
**Pagenberg-Dost-Altenburg & Partner Postfach**
**86 06 20**
**D-8000 München 86 (DE)**

# 0 126 394

**Description**

This invention relates to the removal of aromatic hydroxy compounds and/or aromatic amines from waste waters by employing the enzymes peroxidase and alcohol oxidase or glucose oxidase.

Aromatic hydroxy compounds and aromatic amines are commonly present in waste waters of a number of industries. Such aromatic hydroxy compounds and aromatic amines can be toxic when present in elevated levels. In addition, aromatic hydroxy compounds and aromatic amines also have a relatively high biological oxygen demand and therefore when present in sufficient concentrations can greatly reduce or deplete the oxygen in a body of water containing them.

Conventional processes for removal of aromatic hydroxy compounds and aromatic amines from industrial waste waters include extraction, adsorption on activated carbon, steam distillation, bacterial and chemical reaction (oxidation), electrochemical techniques and irradiation. All of these methods, although certainly feasible and useful, suffer from serious drawbacks, e.g., high cost, incompleteness of purification, formation of hazardous by-products or low efficiency. This situation has necessitated a search for alternative methods.

Recently as disclosed in the *Journal of Applied Biochemistry* 2, pp 414—421 (1980), phenols and anilines present in waste water could be removed by the additon of peroxidase and hydrogen peroxide to the waste water, thereby causing the precipitation of phenols and aromatic amines from the water in the form of insoluble, apparently non-toxic polymers.

The above system, although effective for its intended purpose, has serious shortcomings which preclude it from being commercially attractive. Namely, hydrogen peroxide is expensive, unstable on storage and short lived in a real waste stream situation where metal salts, sunlight, and bacteria break it down rather quickly to oxygen and water. Thus a method which would avoid problems previously experienced is highly desirable.

It is an object of this invention to provide an improved process for the removal of aromatic hydroxy compounds or aromatic amines from waste streams.

Other aspects, objects, and advantages of the present invention will become apparent from this specification and claims.

In accordance with the present invention, it has been discovered that water soluble aromatic hydroxy compounds and/or aromatic amines may be efficiently and economically removed from bodies of water containing the same by treating the water with peroxidase and at least one selected from the group consisting of alcohol oxidase and a straight chain $C_1$ to $C_4$ alcohol or glucose oxidase and glucose.

By treating the water in this manner, $H_2O_2$ is produced continuously *in situ* thereby avoiding the problem of $H_2O_2$ storage instability and thus a constant source of $H_2O_2$ is provided by the reaction of alcohol oxidase or glucose oxidase, their individual substrate, and oxygen.

In the present invention, the term aromatic hydroxy compound is defined as being one wherein an OH group is attached to an aromatic or substituted aromatic nucleus. The aromatic nucleus may be either a monoaromatic compound such as phenol or a polynuclear aromatic compound such as 2-naphthol. Examples of suitable substituent groups on the aromatic nucleus include OH, SH, OR, SR, RSO, $RSO_2$, wherein R is a $C_1$ to $C_{20}$ hydrocarbyl radical, $C_1$ to $C_{20}$ hydrocarbyl radicals themselves, halogens, $SO_3M$ wherein M is hydrogen or a Group IA metal, amides, amines, carboxylic acid and cyano functionalities.

Representative examples of such aromatic hydroxy compounds include phenol, guaiacol, cresol, resorcinol, chlorophenols, aminophenols, 2',7'-dichlorofluorescein, 5,7-dichloro-8-hydroxyquinoline, 1,8-dihydroxyanthraquinone, 2,4-dihydroxy-5,6-dimethylpyrimidine, 4,6-dihydroxy-2-mercaptopyrimidine, 3,6-dihydroxypyridazine, 4,8-dihydroxyquinoline-2-carboxylic acid, 2,3-dihydroxyquinoxaline, 1,5-di-hydroxy-1,2,3,4-tetrahydronaphthalene, 2-(dimethylaminomethyl)-3-hydroxypyridine, 1-naphthol, 1,3-naphthalenediol, 1,2-nitroso-1-naphthol, 2,7-naphthalenediol, p-phenylphenol, 5-indanol, and 8-hydroxyquinoline.

As used in this invention, the term aromatic amine is defined to be one wherein a primary amine group is attached to an aromatic or substituted aromatic nucleus. The aromatic nucleus may be either monoaromatic as in the case of aniline or polynucleus as in the case of 1-aminonaphthalene. Examples of possible substitution groups on the aromatic nucleus are the same as given earlier for the aromatic hydroxy compounds.

Representative examples of such aromatic amines include aniline, benzidine, 4-chloroaniline, 4-bromoaniline, 4-fluoroaniline, 4-bromo-2-methylaniline, m-phenylenediamine, N(1-naphthyl)ethylenediamine, 1-aminonaphthalene, 2-aminonaphthalene, ethidium bromide, 6-hydroxy-2,4,5-triaminopyrimidine sulfate, N'-(6-indazolyl)sulfanilamide, 5'-iodo-5'-deoxyadenosine, o-dianisidine, 3,3'-diaminobenzidine, 3,3'-dichlorobenzidine, o-tolidine, p-phenylazoaniline, 4-aminophenyl, 1-naphthylamine, 2-naphthylamine, and 5-nitro-1-naphthylamine.

Whatever aromatic hydroxy compound or aromatic amine are removed must have generally a solubility in water of at least about 0.01 mg/liter, and preferably at least about 0.05 mg/liter.

Peroxidase, alcohol oxidase, and glucose oxidase enzymes are commercially available from biological supply houses such as Sigma Chemical Company, St. Louis, U.S.A.

Preferably the alcohol oxidase utilized in this invention is purified from *Pichia pastoris* which is known

2

to have low sensitivity to $H_2O_2$. The purified alcohol oxidase employed is preferably essentially free of catalase activity.

A suitable glucose oxidase utilized in this invention is purified from *Aspergillus niger*.

Suitable sources of peroxidase enzyme are purified from, plant, animal and microbial sources. Included are horseradish peroxidase, turnip peroxidase, seaweed peroxidase, chloroperoxidases such as isolated from *Caldariomyces fumago* and lactoperoxidases such as isolated from mammalian milk.

The alcohol substrates useful in the present invention are the straight chain $C_1$ to $C_4$ alcohols. Most commonly used are methanol and ethanol and most preferred is methanol.

Suitable substrates for glucose oxidase include β-D-glucose, also known as dextrose.

In addition, the glucose oxidase substrate can be indirectly provided to the reaction mixture by employing a precursor carbohydrate in combination with an appropriate hydrolase enzyme. For example, starch can be hydrolyzed to glucose in the presence of amylase and glucoamylase; sucrose can be converted to a mixture of glucose and fructose in the presence of invertase; lactose can be converted to glucose and galactose in the presence of lactase; cellulose can be converted to glucose in the presence of cellulase.

The amount of peroxidase and oxidase enzymes added to the body of water is not generally dependent upon the concentration of aromatic hydroxy compound or aromatic amine present. The more critical variable appears to be the amount of oxidase enzyme substrate employed, since the amount of $H_2O_2$ which can ultimately be generated will be limited by the amount of substrate provided. Thus, the greater the concentration of aromatic hydroxy compound or aromatic amine in the solution to be treated, the greater amount of oxidase substrate will preferably be employed. The order of addition of these reagents to the reaction mixture is not thought to be critical in the present invention.

The following Table sets forth the ranges of reagents per liter of waste water which are believed useful:

TABLE I

| Reagent | Concentration | |
| --- | --- | --- |
| | Broad | Preferred |
| Oxidase substrate | 5—10,000 mg/L | 25—750 mg/L |
| Oxidase enzyme | 0.1—10,000 U/L | 10—1000 U/L |
| Peroxidase | 0.1—10,000 U/L | 10—1000 U/L |

An enzyme unit (U) is described as that quantity of enzyme which catalyzes the transformation of 1 µmole of substrate per minute under standard conditions. Whatever concentration of reagents are employed, they should be present in an amount sufficient to effectively remove all or essentially all of the aromatic hydroxy compounds and/or aromatic amines present.

In a presently preferred embodiment of the present invention, sodium azide is added to a concentrated stock solution of peroxidase and either alcohol oxidase or glucose oxidase. The azide ion stabilizes the enzyme by physical interactions with the proteins. Therefore it is contemplated that the use of sodium azide in the present invention will operate to stabilize the combination of peroxidase and either alcohol oxidase or glucose oxidase enzymes. Generally, the $NaN_3$ in concentrated stock solutions will be present in an amount from about 100 to over 500 mg/L, preferably about 200 mg/L. Upon addition of a concentrated enzyme stock solution to the waste water, the azide salt is greatly diluted out and is no longer bacteriostatic or inhibitory to the enzymes.

Generally, the reaction temperature should be from 0°C to 50°C, preferably 20°C to 45°C.

Generally, the reaction should be conducted at a pH in the range of 4—11 and preferably 6—10.

Generally, reaction or treatment time will be from 5 min. to 48 hours.

Generally, the reaction should be aerated by stirring or bubbling air through the solution.

At the end of the reaction time, the precipitated aromatic hydroxy compounds and aromatic amines may be separated from the water containing them by such conventional techniques as filtration, centrifugation, sedimentation or flotation. Since the polymerized end products appear to be non-toxic, they may also be left in the treated stream.

Immobilized enzymes are also operable in the practice of the present invention. Thus, peroxidase and an oxidase enzyme can be immobilized and loaded into a flow reactor through which the fluid to be treated is then passed. Such a mode of operation has the benefit of allowing continuous wastewater treatment and reuse of the enzymes.

The following examples further illustrate the present invention.

Example I
Alcohol oxidase preparation and purification
In a continuous aerobic fermentation process, methanol and an aqueous mineral salts medium in a volume ratio of 40 to 60, respectively, were fed individually to a fermenter, inoculated with the yeast

3

species Pichia pastoris NRRL Y-11430, at a rate so that methanol is the growth-limiting factor. The fermenter was a 1500-liter foam-filled fermenter with a liquid volume of about 610 liters, with automatic pH, temperature, and level control. Agitation was provided by two conventional paddle-type turbines driven at 1000 rpm. The aeration rate was about 4 volumes of air (at about 38 psig (262 kPa) and about 25°C) per volume of ferment in the fermenter per minute. Anhydrous ammonia was added at such a rate as to maintain the pH of the fermentation mixture at about 3.5.

The aqueous mineral salts medium was prepared by mixing, with each liter of tap water, 15.86 mL 75 percent $H_3PO_4$, 9.53 g $K_2SO_4$, 7.8 g $MgSO_4 \cdot 7H_2O$, 0.6 g $CaCl_2 \cdot 2H_2O$, and 2.6 g 85 percent KOH. The trace mineral solution plus biotin was fed separately via the methanol stream at a rate of 10 mL per liter of methanol. The trace mineral solution plus biotin was prepared by mixing 780 mL of a trace mineral solution, 20 mL water, 200 mL methanol and 0.032 g biotin.

The trace mineral solution was prepared by mixing, for each liter of solution, 65 g $FeSO_4 \cdot 7H_2O$, 20 g $ZnSO_4 \cdot 7H_2O$, 3.0 g $MnSO_4 \cdot H_2O$, 6.0 g $CuSO_4 \cdot 5H_2O$, 5.0 mL conc. $H_2SO_4$, and sufficient deionized water to make 1 liter of solution.

The aqueous mineral salts medium was fed at a rate of 31.5 liters per hour and the methanol at a rate of 21 liters per hour.

The fermentation was conducted at about 30°C and about 38 psig (262 kPa) pressure, with a fermentation time of 11.6 hours.

For analytical purposes, the resulting yeast cells were separated from the fermentation effluent (ferment) by centrifugation, washed by suspension in water and recentrifugation, dried overnight at 100°C, and weighed. On a dried basis, the yield of yeast cells typically was about 40.6 g per 100 g of methanol fed. The cell density typically was about 128.4 g of cells per liter of fermenter effluent. The total solids content of the ferment typically was about 134.7 g per liter, cells plus dissolved solids. A portion of the fermenter effluent was frozen and stored.

Fermentation of Pichia pastoris NRRL Y-11430 was carried out by a method of which that set forth above is typical. A portion of the fermenter effluent was removed and adjusted to pH 7.5 with ammonium hydroxide, and was homogenized on a Dyno-Mill® Model KDL using a 0.6 liter vessel in a continuous operation at 30°C using belt combination #3 and a flow of 20—30 mL/hr. The beads in the mill were lead-free glass beads with a diameter of 0.3—0.5 mm. The resulting homogenate was centrifuged at 5°C and 20,000×g for 30 minutes to yield a cell-free supernatant. The cell-free supernatant enzyme activity measured by the dye-peroxidase method described below was about 330 EU/mL. The supernatant was stored frozen for future use. This describes the preparation of crude yeast homogenate.

Six 130 mL portions of the supernatant were placed in cellulose acetate dialysis bags and dialyzed at 5°C against about 8 litres of distilled water. After 4 days, the aqueous phase of each bag was decanted. The solids remaining in the bags consisted of two types of solid. The thin upper white layer was carefully removed and discarded. The bottom solid was brown-yellow and was crystalline alcohol oxidase. A portion of the crystalline alcohol oxidase was dissolved in distilled water (about 10 times the volume of the solid) and an assay by the dye-peroxidase method described below showed an activity of 94 EU/mL. The specific activity of the alcohol oxidase was 10.4 EU/mg of protein.

A sample of the solid alcohol oxidase was examined by SDS gel electrophoresis and a single band was observed indicating a homogeneously pure enzyme. A comparison of electrophoretic mobility with those of proteins having known molecular weight indicates a subunit molecular weight of about 72,000±3000 (estimated). This describes the preparation of pure yeast alcohol oxidase from Pichia pastoris.

The alcohol oxidase activity for reaction with methanol was determined by the dye-peroxidase method. A dye-buffer mixture was prepared by mixing 0.1 mL of an o-dianisidine solution (1 weight percent o-dianisidine in water) with 12 mL of aerated 0.1 M sodium phosphate buffer (pH 7.5). The assay mixture was prepared with 2.5 mL of the dye-buffer mixture, 50 μL of methanol, 10 μL of a peroxidase solution (1 mg of horse-radish peroxidase-Sigma, Type II), and 25 μL of the alcohol oxidase solution. The assay mixture was maintained at 25°C in a 4×1×1 cm cuvette and the increase in absorbance by the dye at 460 nm was recorded for 2 to 4 minutes. The enzyme activity was calculated by

$$\text{Activity (} \mu \text{ mole/min/mL or Enzyme Units/mL)} = \frac{\Delta A}{\text{min.}} \times 11.5$$

wherein 11.5 is a factor based on a standard curve prepared with known aliquots of $H_2O_2$ and $\Delta A$ is the change in absorbance during the experimental interval.

Example II

The prior art treatment of water containing phenols was repeated as follows. A 250 mL beaker was charged with 100 mL of 50 m$M$ sodium acetate buffer (pH 3.5 or 5.5) or 100 mL of 10 m$M$ potassium phosphate buffer (pH 7.5), 1 mL of horseradish peroxidase (100 U/mL), 10 μL of water-saturated phenol or guaiacol (2-methoxyphenol) and 10 μL of 30% aqueous $H_2O_2$. The mixture was incubated at room temperature for 3 hours without stirring. Phenol removal was determined by gas liquid chromatography (glc), results presented below.

4

**0 126 394**

| Sample | Phenol employed | pH | Phenol removal |
|--------|-----------------|-----|----------------|
| 1 | Phenol | 3.5 | 52% |
| 2 | Guaiacol | 5.5 | 100% |
| 3 | Guaiacol | 7.5 | 93% |

A similar mixture was prepared in a 1000 mL beaker employing alcohol oxidase plus alcohol in place of the $H_2O_2$. 100 mL of 50 m$M$ potassium phosphate buffer (pH 7.5), 1 mL of horseradish peroxidase (100 U/mL), 10 µL of guaiacol, 100 µL of alcohol oxidase (AO) solution (1000 U/mL) and 100 µL of methanol or ethanol were mixed, then stirred for 3 hours at room temperature. Phenol removal was determined as above. Guaiacol removal after three hours was essentially quantitative (~100%) when either methanol or ethanol was employed.

The results of these experiments demonstrate that the inventive waste water treatment process is at least as effective as prior art enzymatic treatment processes.

Example III

The effect of alcohol oxidase concentration on the percent removal of aromatic hydroxy compound was studied. A standard mixture containing 100 mL of 50 m$M$ potassium phosphate buffer (pH 7.5), 1 mL of peroxidase (100 U/mL), 10 µL of phenol (water saturated) or guaiacol, and 100 µL of methanol in a 1000 mL beaker was treated with aliquots of alcohol oxidase (1000 U/mL total), then stirred at room temperature for 3 hours before being analyzed by glc for phenol or guaiacol removal.

| Sample | µL added AO | % Phenol removal | |
|--------|-------------|----------|--------|
| | | Guaiacol | Phenol |
| 1 | 0 | 0 | 0 |
| 2 | 1 | 89.5 | 83 |
| 3 | 5 | 98.6 | 88 |
| 4 | 25 | 98.6 | 96 |
| 5 | 100 | — | 99.8 |

The results of these experiments demonstrate the effectiveness of the inventive process for the removal of aromatic hydroxy compounds such as guaiacol from an aqueous solution. Excellent aromatic hydroxy compound removal is achieved even with very low levels of alcohol oxidase (~1U AO/100 mL of solution).

Example IV

The rate of aromatic hydroxy compound removal from an aqueous solution was studied by following the disappearance of guaiacol as a function of time for the following reagent mixture:

10 µL guaiacol
100 mL 50 m$M$ potassium phosphate buffer (pH 7.5)
1 mL horseradish peroxidase (100 U/mL)
5 µL alcohol oxidase (1000 U/mL)
100 µL methanol

This mixture was stirred in a 1000 mL beaker at room temperature, and monitored from time to time by glc for guaiacol disappearance.

| Sample | Time, hr | % Guaiacol removal |
|--------|----------|--------------------|
| 1 | 0 | 0 |
| 2 | 0.2 | 51 |
| 3 | 0.4 | 77 |
| 4 | 1 | 98.3 |
| 5 | 2 | 98.3 |

5

The results of these experiments demonstrate that aromatic hydroxy compound removal by the inventive method is quite rapid-over half of the guaiacol having been removed in only 12 minutes (Sample 2), with essentially complete guaiacol removal after only one hour (Sample 4).

Example V

The effect of pH on the efficacy of the inventive waste water cleanup system was investigated over a wide pH range. A 1000 mL beaker was charged with:

    10 µL of water saturated phenol
    100 µL of methanol
    200 µL of alcohol oxidase (1000 U/mL)
    1 mL horseradish peroxidase (100 U/mL)
and either:
    100 mL of 50 mM sodium acetate buffer (pH 4—6) or
    100 mL of 50 mM potassium phosphate buffer (pH 7—11)

The various mixtures were stirred at room temperature for three hours, then monitored by glc for phenol removal.

| Sample | pH | % Phenol removal |
|--------|----|-----------------|
| 1 | 4 | 19 |
| 2 | 5 | 61 |
| 3 | 6 | 95.4 |
| 4 | 7 | 95.4 |
| 5 | 8 | 98.6 |
| 6 | 9 | 99.1 |
| 7 | 10 | 98.9 |
| 8 | 11 | 26.6 |

The results of these experiments demonstrate that the inventive waste water treatment process is effective over the pH range of 4—11. Particularly good results are obtained in the pH range of 6—10. At a pH of 10, the solids formed in the presence of peroxidase, methanol and alcohol oxidase aggregated to the largest particle size thus indicating the preferred pH where solids removal would also be desired.

Example VI

The effect of methanol concentration on the degree of aromatic hydroxy compound removal was studied by adding various amounts of methanol to a 1000 mL beaker containing:

    100 mL 50 mM potassium phosphate buffer (pH 7.5)
    1 mL horseradish peroxidase (100 U/mL)
    200 µL alcohol oxidase (1000 U/mL)
    10 µL guaiacol

The resulting mixture was stirred at room temperature for three hours, and the monitored by glc for guaiacol removal.

| Sample | Added methanol, µL | % Guaiacol removal |
|--------|-------------------|-------------------|
| 6 | 0 | 0 |
| 7 | 1 | 60 |
| 8 | 5 | 97.9 |
| 9 | 25 | 98.6 |
| 10 | 100 | 99.2 |

The procedure employed above was then repeated, except only 10 µL of alcohol oxidase were used.

6

| Sample | Added methanol, μL | % Guaiacol removal |
|--------|--------------------|--------------------|
| 1 | 0 | 0 |
| 2 | 1 | 47 |
| 3 | 5 | 98.4 |
| 4 | 25 | 99.3 |
| 5 | 100 | 99.5 |

The results of these experiments indicate that effective aromatic hydroxy compounds removal is achieved with the inventive waste water treatment process with very low levels of alcohol such as methanol present. Thus, greater than 98% guaiacol removal was observed when only 10 μL of alcohol oxidase and 5 μL of methanol were added to 100 mL of water containing a phenolic compound.

Example VII

The effect of peroxidase concentration on the efficiency of aromatic hydroxy compound removal was studied. A 1000 mL beaker was charged with 100 mL 50 m$M$ potassium phosphate buffer (pH 7.5), 10 μL of alcohol oxidase (1000 U/mL), 10 μL of methanol, 10 μL of guaiacol or phenol, and 0—1 mL of peroxidase (100 U/mL). The mixtures were stirred at room temperature for three hours, then monitored by glc for disappearance of aromatic hydroxy compound.

| | | % Aromatic hydroxy compound removal | |
|--------|-----------------------------------|----------|----------|
| Sample | μL Added horseradish peroxidase | Guaiacol | Phenol |
| 1 | 0 | 0 | 0 |
| 2 | 1 | 8 | 4 |
| 3 | 5 | 15 | 0 |
| 4 | 25 | 73 | 16 |
| 5 | 50 | 96.7 | — |
| 6 | 100 | 97.6 | 24.4,45* |
| 7 | 200 | 99.6 | 59* |
| 8 | 500 | 99.6 | 83* |
| 9 | 1000 | 100 | 99.9* |

*100 μL methanol employed

The results of these experiments demonstrate that effective aromatic hydroxy compound removal can be achieved employing very low levels of horseradish peroxidase—as low as 25 μL per 100 mL of aromatic hydroxy compounds containing solution. Excellent results are obtained employing about 100 U/L of horseradish peroxidase for guaiacol removal and about 1000 U/L of horseradish peroxidase for phenol removal.

Example VIII

The applicability of the inventive waste water treatment method to 1-naphthol and benzidine was studied. A 1000 mL beaker was charged with 100 mL of 50 m$M$ potassium phosphate buffer (pH 7.5), 100 μL of methanol containing 10 mg of 1-naphthol or benzidine, and amounts of horseradish peroxidase solution (100 U/mL) and alcohol oxidase solution (1000 U/mL) as indicated below. Samples were stirred at room temperature for seven hours. 1-Naphthol removal was monitored by glc, benzidine removal was monitored by measuring the absorbancy at 280 nm of the sample and comparing to a standard curve.

# 0 126 394

| | | | % Removal | |
|---|---|---|---|---|
| Sample | µL Peroxidase added | | 1-Naphthol | Benzidine |
| **A. 100 µL A0** | | | | |
| 1 | 0 | | 0 | 0 |
| 2 | 1 | | 0 | 0 |
| 3 | 2 | | 5.5 | 36 |
| 4 | 5 | | 11.4 | 10 |
| 5 | 10 | | 19.0 | 45 |
| 6 | 25 | | 39.8 | 65 |
| 7 | 50 | | 58.6 | 71 |
| 8 | 100 | | 83.6 | 100 |
| **B. 10 µL A0** | | | | |
| 9 | 0 | | 0 | 0 |
| 10 | 25 | | 12 | 20.1 |
| 11 | 50 | | 36 | 27.4 |
| 12 | 100 | | 58.8 | 58.0 |
| 13 | 250 | | 100 | 100 |

The results of these experiments demonstrate that the inventive waste water treatment process is effective for removing a variety of compounds from water, such as 1-naphthol and benzidine. In addition, it is shown that alcohol oxidase levels as low as 10 µL/100 mL (or about 10 units of enzyme per liter of water to be treated) and peroxidase levels as low as about 50 µL/100 mL (or about 50 enzyme units per liter of water to be treated) can be employed.

Example IX

The rate of benzidine removal from an aqueous solution was studied by measuring the disappearance of benzidine from a solution containing:

100 mL 50 m$M$ potassium phosphate buffer (pH 7.5)
100 µL alcohol oxidase (1000 U/mL)
100 µL horseradish peroxidase (100 U/mL)
100 µL methanol
10 mg benzidine

The solution was stirred at room temperature in a 1000 mL beaker. Periodically samples were withdrawn for a UV to determination of the amount of benzidine removal accomplished.

| Sample | Time, minutes | % Benzidine removal |
|---|---|---|
| 1 | 0 | 0 |
| 2 | 5 | 74 |
| 3 | 30 | 94.7 |
| 4 | 60 | 96.1 |
| 5 | 180 | 97.1 |

The results of these experiments demonstrate that a substantial amount of an aromatic amine compound such as benzidine is rapidly removed by the inventive waste water treatment process, i.e. greater than 70% removal is only 5 minutes.

8

Example X

The effect of added methanol and formaldehyde on the efficiency of the prior art waste water treatment process employing horseradish peroxidase plus $H_2O_2$ was studied. A 1000 mL beaker was charged with 100 mL of 50 m$M$ potassium phosphate buffer (pH 7.5), 1 mL horseradish peroxidase (100 U/mL), 10 µL phenol and various additives as indicated below. The solutions were stirred at room temperature for three hours, then assayed by glc for disappearance of phenol.

| Sample | Additive(s) | % Phenol removal |
|---|---|---|
| 1 | 10 µL $H_2O_2$ (30%) | 41.4 |
| 2 | (1)+300 µL HCHO (37%) | 37.2 |
| 3 | (1)+100 µL MeOH | 52.7 |
| 4* | 100 µL MeOH+10 µL AO (1000 U/mL) | 61.1 |
| 5** | 10 µL $H_2O_2$ | 0 |

\*$H_2O_2$ omitted from this sample
\*\*Peroxidase omitted from this sample

Sample 4 indicates that the addition of methanol and alcohol oxidase, thereby resulting in the *in situ* production of $H_2O_2$, resulted in the highest removal of phenol. Sample 1 corresponds to the prior art treatment of waste water wherein $H_2O_2$ is added directly. Samples 2 and 3 show that the mere addition of formaldehyde and methanol in combination with $H_2O_2$ to the waste water treatment process can result in detrimental effects (Sample 2) or beneficial effects (Sample 3) which, however, is still below the results achieved by the process of the present invention (Sample 4).

Example XI

Additional experiments were carried out to determine the effect of added methanol and formaldehyde on the prior art waste water treatment process. Thus, a 1000 mL beaker containing 100 mL 10 m$M$ potassium phosphate buffer (pH 7.0), 10 µL phenol, 100 µL horseradish peroxidase, and additional reagents as indicated below was stirred at room temperature for three hours, then assayed by glc for disappearance of phenol.

| Sample | Additive(s) | % Phenol removal |
|---|---|---|
| 1* | None | 0 |
| 2 | 10 µL $H_2O_2$ (30%) | 52 |
| 3 | (2)+15 µL HCHO (37%) | 49 |
| 4 | (2)+100 µL MeOH | 53 |
| 5 | (2)+15 µL HCHO+100 mL MeOH | 43 |
| 6 | 10 µL AO (1000 U/mL)+100 µL MeOH | 58 |

\*Peroxidase omitted from this sample

Sample 2 corresponds to the prior art waste water treatment process. As seen in Example X, addition of formaldehyde appears to have a small detrimental effect on the efficiency of the process, and addition of methanol appears to have a small beneficial effect on the efficiency of the process. Run 5, however, demonstrates that simply adding methanol and formaldehyde has a negative effect on the overall treatment process. Compare this to run 6 where methanol and alcohol oxidase are added, thereby leading to the production of HCHO and $H_2O_2$ in the reaction mixture. The % phenol removal observed is higher than that achieved with any of the reagent combinations with the prior art system.

Example XII

A suitable reagent pack for use in the inventive waste water treatment process will preferably include the presence of a preservative and enzyme stabilizer. The following experiment was carried out to

9

# 0 126 394

demonstrate that an azide compound such as $NaN_3$ could be employed to preserve an alcohol oxidase/horseradish peroxidase enzyme combination without irreversibly inhibiting the enzymes. The dye-peroxidase assay procedure described in Example I was carried out on two samples, one containing 0.02 wt.% $NaN_3$ and the control sample containing no $NaN_3$. The change in absorbance, $\Delta A$, for the two samples were essentially the same (53.8 in the presence of $NaN_3$, 54.0 absent $NaN_3$).

This experiment demonstrates that sodium azide is a reversible inhibitor for the enzyme combination alcohol oxidase/peroxidase.

Example XIII

The inventive waste water treatment process was compared to a bacterial process for phenol removal. For reactor cells (300 mL) were charged with 100 mL of solution containing 35 mg/L of phenol. These cells were further treated as follows:

1) Vigorous air sparging only;
2) Peroxidase, alcohol oxidase, methanol and vigorous air sparging,
3) As in (2) plus an inoculum of bacteria acclimated to a continuous feed of 3.5 mg/L of phenol;
4) An inoculum of bacteria as employed in (3) with vigorous air sparging.

The peroxidase and alcohol oxidase were added in (2) and (3) at the rate of 1 mL of a stabilized enzyme solution (comprising 1000 U of alcohol oxidase, 1000 U of horseradish peroxidase, and 2 mg of $NaN_3$ in 10 mL of 50 m$M$ potassium phosphate buffer (pH 7.5)) per liter of solution to be treated. Methanol was added separately at the rate of 0.1 mL/L of solution to be treated.

The four samples were sparged vigorously with air for three hours at room temperature, then analyzed for phenol content.

| Sample | Additive(s) | % Phenol removal |
|--------|-------------|------------------|
| 1 | None | 2.7 |
| 2 | Peroxidase, A0, MeOH | 100 |
| 3 | Peroxidase, A0, MeOH, bacteria | 99.7 |
| 4 | Bacteria | 10.3 |

The results of these experiments demonstrate that the inventive waste water treatment process is effective for removing phenol from a simulated waste stream (See Sample 2). Note that bacteria adapted to growth on lower levels of phenol impurity are relatively ineffective over the time period studied. Further, the combination of enzymatic treatment disclosed herein with bacterial phenol removal is seen to be compatible, such that the inventive method would provide a suitable means to treat surges of waste water impurities.

Example XIV

The efficiency of the inventive wastewater treatment process compared to the prior art method was tested as follows. A 1000 mL beaker was charged with:

100 mL of 50 m$M$ potassium phosphate buffer (pH 7.5)
1 mL of horseradish peroxidase (100 U/mL)
10 µL of water-saturated phenol
and either:
400 µL of 30% $H_2O_2$
or
100 µL methanol plus
100 µL of alcohol oxidase (100 U/mL)

Based on the known activity of alcohol oxidase, the methanol-alcohol oxidase combination employed was calculated to be capable of generating the equivalent of 400 µL of 30% $H_2O_2$ at room temperature in one hour. Thus, equivalent amounts of $H_2O_2$ were added to the two samples either directly (prior art) or indirectly (generated *in situ*). Each solution was stirred at room temperature for one hour, then assayed for disappearance of phenol. The prior art sample (direct addition of $H_2O_2$) gave 50% phenol removal while the inventive treatment (*in situ* $H_2O_2$ generation) gave essentially quantitative phenol removal over the same time period.

The results of these experiments demonstrate the surprising improvement in the efficiency of removal of aromatic hydroxy compounds by *in situ* generation of $H_2O_2$ versus direct addition of $H_2O_2$ in the presence of peroxidase enzyme.

Example XV

The inventive wastewater treatment process was carried out employing peroxidase, glucose oxidase and a suitable substrate. Thus, a 1000 mL beaker was charged as follows:

10

**0 126 394**

100 mL of 0.02 $N$ sodium acetate buffer (pH 5.5)
100 U peroxidase (Sigma Chemical Co., Type II)
130 U glucose oxidase (Sigma Chemical Co., Type VII)
10 µL guaiacol

In one run, 0.5 g of D-glucose was added as substrate for glucose oxidase, while in the other run, 1 g of sucrose and 370 U of invertase (Sigma Chemical Co.) were employed as glucose oxidase substrate. Both samples were stirred at room temperature for three hours, then monitored for guaiacol removal. In both cases, essentially quantitative guaiacol removal was achieved.

The results of these experiments demonstrate that the inventive wastewater treatment process is operable employing glucose oxidase plus glucose or a glucose precursor for *in situ* $H_2O_2$ generation.

Example XVI

The effect of glucose oxidase concentration on the % removal of aromatic hydroxy compounds was studied. Varying amounts of glucose oxidase (130 U/mL) were added to a 1000 mL beaker containing a standard mixture comprising:

100 mL of 20 m$M$ sodium acetate buffer (pH 5.5)
1 mL peroxidase (100 U/mL)
10 µL of guaiacol
0.5 g D-glucose

The solutions were stirred for one hour at room temperature before being analyzed by glc for guaiacol removal.

| Sample | µL Added glucose oxidase | % Guaiacol removal |
|--------|--------------------------|--------------------|
| 1 | 10 | 36 |
| 2 | 25 | 67 |
| 3 | 50 | 100 |
| 4 | 100 | 100 |
| 5 | 250 | 100 |
| 6 | 500 | 100 |
| 7 | 1000 | 100 |

The results of these experiments demonstrate the effectiveness of the inventive process for the removal of aromatic hydroxy compounds such as guaiacol from aqueous solution. Excellent guaiacol removal is achieved with glucose oxidase levels as low as about 50 U per liter of solution to be treated.

**Claims**

1. A process for the removal of at least one compound selected from (a) aromatic hydroxy compounds and (b) aromatic amines having a water solubility of at least about 0.01 mg/l in water containing the same, said aromatic hydroxy compound being one wherein an OH-group is attached to an aromatic or substituted aromatic nucleus, and said aromatic amine being one wherein a primary amine group is attached to an aromatic or substituted aromatic nucleus, wherein said aromatic nucleus is each either mononuclear or polynuclear, characterized by treating the water with peroxidase, and at least one reagent selected from (c) alcohol oxidase and a straight chain $C_1$ to $C_4$ alcohol and (d) glucose oxidase and glucose.

2. The process of claim 1 characterized in that said aromatic hydroxy compounds (a) or aromatic amines (b) have a water solubility of at least about 0.05 mg/liter.

3. The process of claim 1 or 2 characterized in that said alcohol is ethanol or preferably methanol.

4. The process of claim 1 for the removal of compound (a) utilizing reagent (c) characterized in that said alcohol is methanol and said aromatic hydroxy compound is guaiacol or phenol.

5. The process of claim 1 for the removal of compound (b) utilizing reagent (c) characterized in that said alcohol is methanol and said aromatic amine is benzidine or aniline.

6. The process of any of the preceding claims characterized in that it is carried out at a temperature from 0 to 50°C; in particular wherein the reaction time is from 5 min. to 48 hours.

7. The process of any of the preceding claims characterized in that it is carried out at a pH range from 6 to 10.

8. The process of any of the preceding claims characterized in that said peroxidase, alcohol, and alcohol oxidase are present, respectively, in amounts of from 0.1—10,000 U 5—10,000 mg, and 0.1—10,000

11

**0 126 394**

U per liter of water, preferably in amounts of from 10—1,000 U, 25—750 mg, and 10—1,000 U per liter of water.

9. The process of any of claims 1 to 7 characterized in that said peroxidase, glucose, and glucose oxidase are present, respectively, in amounts of from 0.1—10,000 U, 5—10,000 mg and 0.1—10,000 U per liter of water, preferably in amounts of from 10—1,000 U, 25—750 mg, and 10—1,000 U per liter of water.

10. The process of any of the preceding claims characterized in that sodium azide is additionally added.

11. An enzyme reagent system for carrying out the process of claim 10, comprising (1) peroxidase, (2) alcohol oxidase or glucose oxidase and (3) sodium azide.

**Patentansprüche**

1. Verfahren zur Entfernung, von mindestens einer Verbindung, ausgewählt aus (a) aromatischen Hydroxyverbindungen und (b) aromatischen Aminen mit einer Wasserlöslichkeit von mindestens etwa 0,01 mg/l in Wasser, welches sie enthält, wobei die aromatische Hydroxyverbindung einen solche ist, bei der eine OH-Gruppe an einen aromatischen oder substituierten aromatischen Kern gebunden ist, und wobei das aromatische Amin ein solches ist, bei dem eine primäre Aminogruppe an einen aromatischen oder substituierten aromatischen Kern gebunden ist, wobei der aromatische Kern jeweils entweder einkernig oder vielkernig ist, dadurch gekennzeichnet, daß man das Wasser mit Peroxidase und mit mindestens einem Reagenz behandelt, das aus (c) Alkoholoxidase und einem geradkettigen $C_1$- bis $C_4$-Alkohol und (d) Glucoseoxidase und Glucose ausgewählt ist.

2. Verfahren nach Anspruch 1, dadurch gekennzeichnet, daß die aromatischen Hydroxyverbindungen (a) oder aromatischen Amine (b) eine Wasserlöslichkeit von mindestens etwa 0,05 mg/Liter aufweisen.

3. Verfahren nach Anspruch 1 oder 2, dadurch gekennzeichnet, daß der Alkohol Ethanol oder, bevorzugt, Methanol ist.

4. Verfahren nach Anspruch 1 zur Entfernung von Verbindungen (a) unter Verwendung von Reagenz (c), dadurch gekennzeichnet, daß der Alkohol Methanol ist und daß die aromatische Hydroxyverbindung Guajakol oder Phenol ist.

5. Verfahren nach Anspruch 1 zur Entfernung von Verbindungen (b) unter Verwendung von Reagenz (c), dadurch gekennzeichnet, daß der Alkohol Methanol ist und daß das aromatische Amin Benzidin oder Anilin ist.

6. Verfahren nach einem der vorhergehenden Ansprüche, dadurch gekennzeichnet, daß man es bei einer Temperatur von 0 bis 50°C ausführt, wobei insbesondere die Reaktionszeit von 5 min. bis 48 Std. beträgt.

7. Verfahren nach einem der vorhergehenden Ansprüche, dadurch gekennzeichnet, daß man es in einem pH-Bereich von 6 bis 10 durchführt.

8. Verfahren nach einem der vorhergehenden Ansprüche, dadurch gekennzeichnet, daß die Peroxidase, der Alkohol und die Alkoholoxidase jeweils in Mengen von 0,1 bis 10.000 U, 5 bis 10.000 mg und 0,1 bis 10.000 U pro Liter Wasser, bevorzugt in Mengen von 10 bis 1.000 U, 25 bis 750 mg und 10 bis 1.000 U pro Liter Wasser anwesend sind.

9. Verfahren nach einem der Ansprüche 1 bis 7, dadurch gekennzeichnet, daß die Peroxidase, Glucose und die Glucoseoxidase jeweils in Mengen von 0,1 bis 10.000 U, 5 bis 10.000 mg und 0,1 bis 10.000 U pro Liter Wasser, bevorzugt in Mengen von 10 bis 1.000 U, 25 bis 750 mg und 10 bis 1.000 U pro Liter Wasser anwesend sind.

10. Verfahren nach einem der vorhergehenden Ansprüche, dadurch gekennzeichnet, daß man zusätzlich Natriumazid zusetzt.

11. Enzymreagenzsystem zur Durchführung des Verfahrens nach Anspruch 10, enthaltend (1) Peroxidase, (2) Alkoholoxidase oder Glucoseoxidase und (3) Natriumazid.

**Revendications**

1. Procédé d'élimination d'au moins un composé choisi parmi (a) des composés hydroxylés aromatiques et (b) des amines aromatiques ayant une solubilité dans l'eau d'au moins environ 0,01 mg/l dans de l'eau contenant ceux-ci, ce composé hydroxylé aromatique étant un composé dans lequel un groupe OH est fixé sur un noyau aromatique ou aromatique substitué, et cette amine aromatique étant une amine dans laquelle un groupe amine primaire est fixé sur un noyau aromatique ou aromatique substitué, dans lequel ce noyau aromatique est soit mononucléaire, soit polynucléaire, caractérisé en ce qu'on traite l'eau par la peroxydase, et au moins un réactif choisi parmi (c) l'alcool oxydase et un alcool à chaîne linéaire en $C_1$ à $C_4$ et (d) la glucose oxydase et le glucose.

2. Procédé de la revendication 1, caractérisé en ce que ces composés hydroxylés aromatiques (a) ou ces amines aromatiques (b) ont une solubilité dans l'eau d'au moins environ 0,05 mg/l.

3. Procédé suivant les revendications 1 ou 2, caractérisé en ce que cet alcool est l'éthanol, ou de préférence le méthanol.

4. Procédé de la revendication 1 pour l'élimination du composé (a) en utilisant le réactif (c), caractérisé en ce que cet alcool est le méthanol et en ce que ce composé hydroxylé aromatique est le guaiacol ou le phénol.

5. Procédé de la revendication 1 pour l'élimination du composé (b) en utilisant le réactif (c), caractérisé en ce que cet alcool est le méthanol et en ce que cette amine aromatique est la benzidine ou l'aniline.

6. Procédé suivant l'une quelconque des revendications précédentes, caractérisé en ce qu'il est effectué à une température de 0 à 50°C, en particulier dans lequel le temps de réaction est de 5 mn à 48 heures:

7. Procédé suivant l'une quelconque des revendications précédentes, caractérisé en ce qu'il est effectué dans un intervalle de pH de 6 à 10.

8. Procédé suivant l'une quelconque des revendications précédentes, caractérisé en ce que cette peroxydase, cet alcool et cet alcool oxydase sont présents respectivement dans des quantités de 0,1—10000 U, 5—10000 mg et 0,1—10000 U par litre d'eau, de préférence dans des quantités de 10—1000 U, 25—750 mg et 10—1000 U par litre d'eau.

9. Procédé suivant l'une quelconque des revendications 1 à 7, caractérisé en ce que cette peroxydase, ce glucose et cette glucose oxydase sont présents respectivement dans des quantités de 0,1—10000 U, 5—10000 mg et 0,1—10000 U par litre d'eau, de préférence dans des quantités de 10 à 1000 U, 25 à 750 mg et 10 à 1000 U par litre d'eau.

10. Procédé suivant l'une quelconque des revendications précédentes, caractérisé en ce qu'on ajoute en outre de l'azide de sodium.

11. Système de réactifs enzymatique pour effectuer le procédé de la revendication 10, comprenant (1) de la peroxydase, (2) de l'alcool oxydase ou de la glucose oxydase et (3) de l'azide de sodium.